(19) Europäisches Patentamt
European Patent Office
Office européen des brevets

(11) **EP 2 221 088 B1**

(12) **EUROPÄISCHE PATENTSCHRIFT**

(45) Veröffentlichungstag und Bekanntmachung des Hinweises auf die Patenterteilung:
**12.10.2011 Patentblatt 2011/41**

(51) Int Cl.:
***A61N 5/10*** *(2006.01)*

(21) Anmeldenummer: **09178624.4**

(22) Anmeldetag: **10.12.2009**

(54) **Optimierung von Steuerparametern für eine Partikelbestrahlungsanlage unter Berücksichtigung der Teilchenzahldynamik**

Optimisation of control parameters for a particle radiation assembly considering particle count dynamics

Optimisation de paramètres de commande pour une installation de rayonnement de particules en prenant en compte la dynamique numéraire des petites pièces

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **24.02.2009 DE 102009010284**

(43) Veröffentlichungstag der Anmeldung:
**25.08.2010 Patentblatt 2010/34**

(73) Patentinhaber: **Siemens Aktiengesellschaft 80333 München (DE)**

(72) Erfinder: **Fieres, Johannes, Dr. 69115, Heidelberg (DE)**

(56) Entgegenhaltungen:
**EP-A1- 1 818 078     US-A1- 2007 201 614**

• **ALEXEI V CHVETSOV ET AL: "Optimization of equivalent uniform dose using the L-curve criterion" PHYSICS IN MEDICINE AND BIOLOGY, TAYLOR AND FRANCIS LTD. LONDON, GB LNKD- DOI:10.1088/0031-9155/52/19/017, Bd. 52, Nr. 19, 21. September 2007 (2007-09-21), Seiten 5973-5984, XP020113097 ISSN: 0031-9155**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft eine Vorrichtung und ein Verfahren zur Bestimmung von Steuerparametern für eine Partikelbestrahlungsanlage. Eine derartige Vorrichtung bzw. ein derartiges Verfahren kann insbesondere im Rahmen der Partikeltherapie Einsatz finden, beispielsweise im Rahmen der Therapieplanung, bei der im Vorfeld einer Bestrahlung Steuerparameter ermittelt werden, die es erlauben, anschließend während der Bestrahlung einen Gegenstand gemäß bestimmten Vorgaben zu bestrahlen.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nichttherapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc. Hierbei werden geladene Partikel wie z.B. Protonen oder Kohlenstoff-oder andere Ionen auf hohe Energien beschleunigt, zu einem Partikelstrahl geformt und über ein Hochenergiestrahltransportsystem zu einem oder mehreren Bestrahlungsräumen geführt. In einem Bestrahlungsraum wird das zu bestrahlende Objekt in einem Zielvolumen mit dem Partikelstrahl bestrahlt.

Aus EP 1 818 078 sind ein Verfahren und eine Vorrichtung zur Bestranlungs planung einer Partikel therapie bekannt, wobei die zu deponierenden Partikelanzanlverteilung unter Verwendung der vorgegebenen Dosis-Verteilung ermittelt wird.

Aus Phys. Med. Biol. 52, pp 5973-5984 und US 2007 0201614 ist es bekannt, die Homogenität der Bestrahlung bei der Planung zu berücksichtigen.

Der Erfindung liegt die Aufgabe zugrunde, eine Vorrichtung und ein Verfahren zur Bestimmung von Steuerparametern für eine Partikelbestrahlungsanlage aufzuzeigen. Ferner sollen ein Verfahren zur Bestrahlung eines Zielvolumens mit Partikeln vorgestellt werden, sowie ein Computerprogramm und ein Computerprogrammprodukt.

Diese Aufgabe wird durch eine Vorrichtung mit den Merkmalen des Anspruchs 1, sowie durch Verfahren, durch ein Computerprogramm und ein Computerprogrammprodukt mit Merkmalen von nebengeordneten Ansprüchen gelöst. Vorteilhafte Ausgestaltungen und Weiterbildungen sind Gegenstand von Unteransprüchen.

Die erfindungsgemäße Vorrichtung dient der Bestimmung von Steuerparametern für eine Partikelbestrahlungsanlage. Hierbei sind mit der Partikelbestrahlungsanlage verschiedene Dosiswerte an unterschiedlichen Zielpunkten in einem Zielvolumen deponierbar, indem von einem Partikelstrahl Abtastpunkte angesteuert werden. Sie umfasst einen Eingang zum Empfangen einer Dosis-Verteilung über Zielpunkte und einen Ermittlungsbestandteil zum Ermitteln einer bei der Bestrahlung zu deponierenden Partikelanzahlverteilung über Abtastpunkte. Die Ermittlung erfolgt hierbei derart, dass sowohl die vorgegebene Dosis-Verteilung verwendet wird, als auch eine Größe, welche Unterschiede zwischen Partikelanzahlen, die der ermittelten Partikelanzahlverteilung entsprechen, verschiedener Abtastpunkte, berücksichtigt.

Die Partikelbestrahlungsanlage kann auf an sich bekannte Weise eine Bestrahlung mit Partikeln durchführen. Sie wird hierbei so gesteuert, dass an verschiedenen Zielpunkten des zu bestrahlenden Volumens eine Dosis deponiert wird, indem eine bestimmte Anzahl von Partikeln einer bestimmten Energie in eine bestimmte Richtung versendet werden. Die Kombination einer bestimmten Partikelenergie mit einer bestimmten Versenderichtung entspricht einem Abtastpunkt. Die Anzahl von Partikeln pro Abtastpunkt ergibt sich aus der ermittelten Partikelanzahlverteilung; die Partikelanzahlverteilung ordnet also zumindest manchen, vorzugsweise allen Abtastpunkten zu, wie viele Teilchen einer bestimmten Energie in die dem Abtastpunkt entsprechende Richtung zu versenden sind. Die Bestimmung der Steuerparameter erfolgt vor der Bestrahlung und dient der effizienten und plangemäßen Durchführung der Bestrahlung.

Zur Ermittlung der gesuchten Partikelanzahlverteilung verwendet die Vorrichtung zumindest zweierlei:

- Eine vorgegebene Dosis-Verteilung:

   Diese ordnet zumindest manchen, vorzugsweise allen,
   Zielpunkten eine Dosis zu, welche sie bei der Bestrahlung erhalten sollen. Bei dieser Dosis handelt es sich z.B. um eine Energiedosis. Sie wird ausgedrückt als eine pro Masse absorbierte Energie. Es ist auch möglich, andere Dosisarten zu verwenden, z.B. eine mit einer biologischen Wirksamkeit gewichtete Energiedosis.

- Eine Größe, welche Unterschiede zwischen Partikelanzahlen verschiedener Abtastpunkte berücksichtigt:

   Bei den Partikelanzahlen handelt es sich um diejenigen,
   welche sich gemäß der ermittelten Partikelanzahlverteilung ergeben. Mittels der Größe werden also verschiedene Abtastpunkte hinsichtlich der für sie bestimmten Partikelanzahl zueinander verglichen.

Die Größe kann die Unterschiede zwischen den Partikelanzahlen von Abtastpunkten auf verschiedene Weisen berücksichtigen. Diesen verschiedenen Möglichkeiten ist gemein, dass zumindest ein Unterschied zwischen einer ersten Partikelanzahl und einer zweiten Partikelanzahl Eingang in die Größe findet, wobei es sich bei diesen Partikelanzahlen auch um Mittelwerte handeln kann.

Von Vorteil ist, dass die genannte Bestimmung von Steuerparametern es einerseits ermöglicht, eine Verkürzung der Behandlungsdauer bei der Bestrahlung zu erreichen. Andererseits ist es hierdurch möglich, den Behandlungsplan robuster gegen Positionsungenauigkeiten zu machen. Denn in der Praxis sind Ungenauigkeiten in der Patientenpositionierung oft unvermeidlich. Dies ist jedoch sehr nachteilig, denn Verschiebungen von 5mm führen i.d.R. schon zu klinisch unakzeptablen Dosisverteilungen. Pläne, die mit der genannten Bestimmung von Steuerparametern erstellt werden, sind deutlich robuster. D.h. die geplante Dosisverteilung kann trotz schlechter Positionierung des Patienten noch relativ gut realisiert werden.

Die Größe berücksichtigt Unterschiede zwischen Partikelanzahlen verschiedener Abtastpunkte, die gemäß der Partikelanzahlverteilung mit Partikeln der gleichen Energie zu bestrahlen sind. Dabei werden mittels der Größe verschiedene Abtastpunkte hinsichtlich der Partikelanzahl verglichen, wobei diese Abtastpunkte alle mit Partikeln der gleichen Energie zu bestrahlen sind. Dies entspricht dem Konzept der Iso-Energieebenen. Der Vergleich der Partikelanzahlen findet also innerhalb einer Iso-Energieebene statt.

In Weiterbildung der Erfindung berücksichtigt die Größe Unterschiede zwischen Partikelanzahlen aller Abtastpunkte, die gemäß der Partikelanzahlverteilung mit Partikeln der gleichen Energie zu bestrahlen sind. Der Vergleich der Partikelanzahlen findet also innerhalb einer Iso-Energieebene statt, wobei alle Abtastpunkte der Ebene in die Größe einbezogen werden.

Einer Ausgestaltung der Erfindung gemäß enthält die Größe eine Streuung von Partikelanzahlen verschiedener Abtastpunkte, die gemäß der Partikelanzahlverteilung mit Partikeln der gleichen Energie zu bestrahlen sind. Der Vergleich der Partikelanzahlen findet also innerhalb einer Iso-Energieebene statt, wobei betrachtet wird, wie stark die Streuung der Werte für die Partikelanzahlen innerhalb der Ebene ist. Hierzu ist es z.B. möglich, als Maß für die Streuung die Varianz zu verwenden. Diese aus der Statistik bekannte Größe wird berechnet, indem die Abstände der Werte vom Mittelwert quadriert, addiert und durch die Anzahl der Werte geteilt werden.

Vorteilhaft ist es ferner, wenn die Größe einen Wertebereich von Partikelanzahlen aller Abtastpunkte, die gemäß der Partikelanzahlverteilung mit Partikeln der gleichen Energie zu bestrahlen sind, enthält. Der Vergleich der Partikelanzahlen findet also innerhalb einer Iso-Energieebene statt, wobei untersucht wird, welcher Wertebereich von den Partikelanzahlen abgedeckt wird. Es ist es z.B. möglich, die maximale Differenz zwischen den Partikelanzahlen verschiedener Abtastpunkte zu betrachten, d.h. die Differenz zwischen dem Abtastpunkt mit der höchsten Partikelanzahl und dem Abtastpunkt mit der niedrigsten Partikelanzahl.

Eine weitere vorteilhafte Möglichkeit ist, dass die Größe das Verhältnis zwischen der höchsten und der niedrigsten Partikelanzahl aller Abtastpunkte, die gemäß der Partikelanzahlverteilung mit Partikeln der gleichen Energie zu bestrahlen sind, enthält. Der Vergleich der Partikelanzahlen findet also innerhalb einer Iso-Energieebene statt, wobei untersucht wird, wie sich die höchste Partikelanzahl innerhalb dieser Ebene zur kleinsten Partikelanzahl innerhalb diese Ebene verhält.

In Bezug auf jede der beschriebenen Möglichkeiten der Ausgestaltung der Größe ist es möglich, dass sie sich nur auf eine bestimmte, auf mehrere, oder auf alle Iso-Energieebenen bezieht.

In Ausgestaltung der Erfindung erfolgt die Ermittlung durch Optimierung einer Kostenfunktion, wobei die Kostenfunktion einen ersten Term betreffend die vorgegebene Dosis-Verteilung enthält, und einen zweiten Term betreffend die Größe. Zur Optimierung von Kostenfunktionen sind zahlreiche Verfahren bekannt, welche sich im Rahmen der Erfindung einsetzen lassen. Vorteilhaft ist es, wenn die Kostenfunktion einen vorgebbaren Parameter enthält zur relativen Wichtung des ersten Terms gegenüber dem zweiten Term. Dieser Parameter kann z.B. wie auch die vorgebbare Dosis-Verteilung über den Eingang der Vorrichtung zur Verfügung gestellt werden. Besonders vorteilhaft ist es, wenn die Optimierung der Kostenfunktion die Partikelanzahlverteilung einerseits hinsichtlich der vorgegebenen Dosis-Verteilung und andererseits hinsichtlich einer Bestrahlungs-Zeitdauer optimiert. Ein Zusammenhang zwischen der Bestrahlungs-Zeitdauer und der Größe ergibt sich insbesondere dann, wenn man von einer zumindest zeitweise konstanten Partikelintensität, d.h. Anzahl ausgestrahlter Partikel pro Zeiteinheit, bei der Bestrahlung ausgeht.

Bei dem erfindungsgemäßen Verfahren zum Bestimmen von Steuerparametern werden Informationen betreffend eine vorgegebene Dosis-Verteilung über Zielpunkte empfangen, wonach eine bei der Bestrahlung zu deponierende Partikelanzahlverteilung über Abtastpunkte ermittelt wird. Die Ermittlung erfolgt unter Verwendung der vorgegebenen Dosis-Verteilung und einer Größe, welche Unterschiede zwischen Partikelanzahlen der Partikelanzahlverteilung verschiedener Abtastpunkte berücksichtigt.

Die erfindungsgemäße Verfahren kann wie obenstehend hinsichtlich der erfindungsgemäßen Vorrichtung erläutert weitergebildet und ausgestaltet werden.

Bei dem erfindungsgemäßen Verfahren zur Bestrahlung eines Zielvolumens mit Partikeln sind die Steuerparameter zur Steuerung der Partikelbestrahlungsanlage wie zuvor beschrieben ermittelt. Es handelt sich bei dem Zielvolumen vollständig oder teilweise um einen nicht-lebenden Körper, z.B. um ein Phantom, welches zur Überprüfung einer Bestrahlungsplanung eingesetzt wird.

Das erfindungsgemäße Computerprogramm verfügt über Programmcode-Mittel, die geeignet sind, ein Verfahren der oben beschriebenen Art durchzuführen, wenn das Computerprogramm auf einem Computer ausgeführt wird.

Das erfindungsgemäße Computerprogrammprodukt umfasst auf einem computerlesbaren Datenträger gespeicherte Programmcode-Mittel, die geeignet sind, ein Verfahren der oben beschriebenen Art durchzuführen, wenn das Computerprogramm auf einem Computer ausgeführt wird.

Im folgenden wird die Erfindung anhand eines Ausführungsbeispiels näher erläutert. Dabei zeigen:

Figur 1: einen schematischen Überblick über den Aufbau einer Partikeltherapieanlage,

Figur 2: eine schematische Darstellung einer Bestrah- lung eines Zielvolumens mittels einer Raster- scaneinrichtung,

Figur 3: eine Teilchenanzahldynamik für zwei verschie- dene ermittelte Partikelanzahlverteilungen,

Figur 4A, 4B: Dosisverteilungen für zwei verschiedene ermit- telte Partikelanzahlverteilungen.

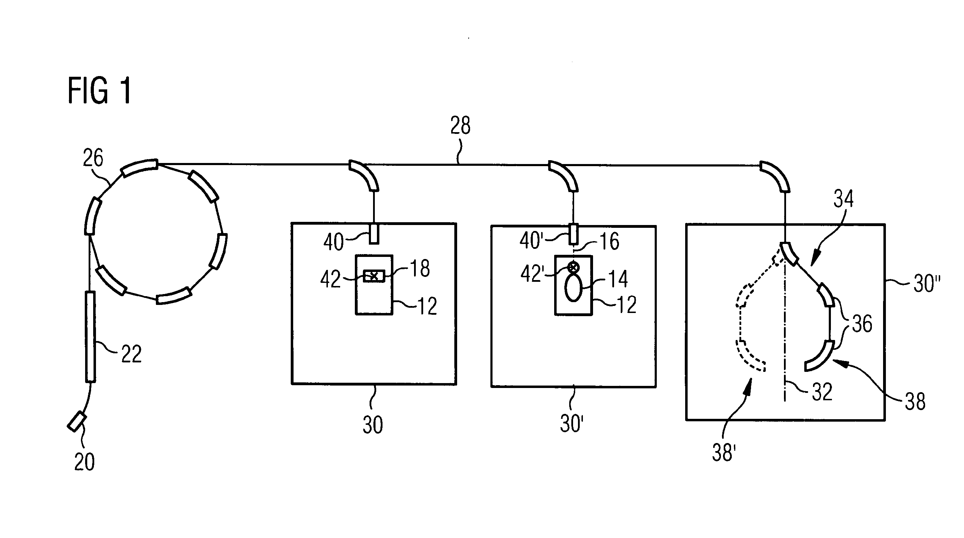

Figur 1 zeigt in schematischer Darstellung einen Aufbau einer Partikeltherapieanlage. Diese wird zur Bestrahlung eines auf einer Positioniervorrichtung 12 angeordneten Patienten 14 mit einem Strahl aus Partikeln 16 eingesetzt, der im Folgenden als Partikelstrahl 16 bezeichnet ist. Insbesondere kann hierdurch ein tumorerkranktes Gewebe des Patienten 14 mit dem Partikelstrahl 16 bestrahlt werden. Es ist ebenfalls möglich, die Partikelbestrahlungsanlage zur Bestrahlung eines nicht-lebenden Objektes 18, insbesondere eines Wasserphantoms 18, einzusetzen. Die Bestrahlung des Wasserphantoms 18 erfolgt beispielsweise zu Zwecken der Überprüfung und Verifizierung von Bestrahlungsparametern vor und/oder nach einer erfolgten Bestrahlung eines Patienten 14. Es kann ferner vorgesehen werden, andere Objektes, insbesondere Versuchsaufbauten wie beispielsweise Zellkulturen oder Bakterienkulturen zu Forschungszwecken mit dem Partikelstrahl 16 zu bestrahlen.

Als Partikel werden vornehmlich Teilchen wie beispielsweise Protonen, Pionen, Heliumionen, Kohlenstoffionen oder Ionen anderer Elemente eingesetzt. Üblicherweise werden derartige Partikel in einer Partikelquelle bzw. Ionenquelle 20 erzeugt. Der von der Ionenquelle 20 erzeugte Ionenstrahl oder Partikelstrahl wird in dem Vorbeschleuniger 22 auf ein erstes Energieniveau beschleunigt. Der Vorbeschleuniger 22 ist beispielsweise ein Linearbeschleuniger. Anschließend werden die Partikel in einen weiteren Beschleuniger 26, beispielsweise einen Kreisbeschleuniger, insbesondere ein Synchrotron oder Zyklotron, eingespeist. In dem Beschleuniger 26 wird der Partikelstrahl auf eine zur Bestrahlung benötigte Energie beschleunigt. Nachdem der Partikelstrahl den Beschleuniger 26 verlassen hat, transportiert ein Hochenergiestrahl-Transportsystem 28 den Partikelstrahl in einen oder mehrere Bestrahlungsräumen 30, 30', 30", wobei dort beispielsweise die Positioniervorrichtung 12 - etwa eine Patientenliege - mit dem Patienten 14 oder das Phantom 18 zur Bestrahlungsplanungsverifikation angeordnet ist. In dem Bestrahlungsraum 30 oder 30' erfolgt die Bestrahlung des Körpers 14, 18 von einer festen Richtung aus, wobei der Körper 14, 18 raumfest angeordnet ist. Diese Bestrahlungsräume 30, 30' werden als "fixed beam"-Räume bezeichnet. Im Behandlungsraum 30" ist eine um eine Achse 32 beweglich angeordnete, vorzugsweise drehbar angeordnete Gantry 34 vorgesehen. Mittels der Gantry 34 kann der zu bestrahlende Körper 14, 18 von verschiedenen Richtungen aus bestrahlt werden. Hierbei wird der Partikelstrahl 16 mittels der in der Gantry 34 angeordneten Gantrystrahlführung 36 um den zu bestrahlenden Körper 14, 18 gedreht. Es sind in Figur 1 stellvertretend für die unterschiedlichen Positionen der Gantrystrahlführung 36 der Gantry 34 eine erste Position 38 und eine zweite Position 38' gezeigt. Selbstverständlich sind auch Zwischenpositionen für die Gantrystrahlführung 36, die aus Gründen der Übersichtlichkeit nicht gezeigt sind, auf zumindest einem Halbkreis oberhalb des zu bestrahlenden Körpers 14, 18 in einer gedachten Kugel um den zu bestrahlenden Körper 14, 18 möglich. Somit kann das zu bestrahlende Zielvolumen von mehreren Richtungen aus senkrecht zur Achse 32 bestrahlt werden. Dies ist aus geometrischen Gründen vorteilhaft.

Im Bestrahlungsraum 30, 30' tritt der Partikelstrahl aus einem als Strahlauslass 40, 40' bezeichneten Ende eines Vakuumsystems der Hochenergiestrahlführung 28 aus und trifft auf das zu bestrahlende Zielvolumen im Körper 14 oder 18. Das Zielvolumen ist hierbei üblicherweise in einem Isozentrum 42, 42' des jeweiligen Bestrahlungsraums 30, 30' angeordnet.

Der anhand der Figur 1 dargestellte Grundaufbau einer Partikeltherapieanlage ist beispielhaft für Partikeltherapieanlagen, kann aber auch hiervon abweichen. Die nachfolgend beschriebenen Ausführungsbeispiele sind sowohl im Zusammenhang mit der anhand von Figur 1 dargestellten als auch mit anderen Partikeltherapieanlagen einsetzbar.

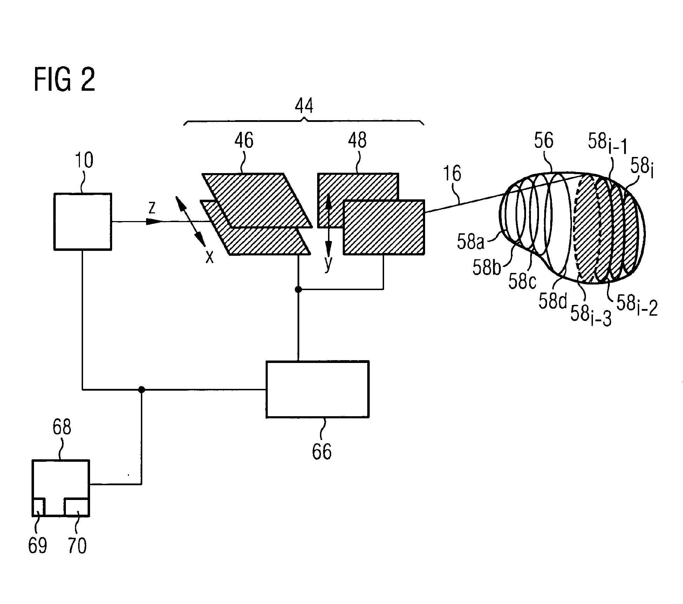

Figur 2 zeigt schematisch eine Bestrahlung eines Zielvolumens 56. Die Rasterscaneinrichtung 44 weist eine erste Partikelstrahlablenkungseinrichtung 46 und eine zweite Partikelstrahlablenkungseinrichtung 48 auf, welche insbesondere Magneten umfassen können. Die beiden Partikelstrahlablenkeinrichtungen 46, 48 vermögen den Strahl 16 horizontal bzw. vertikal abzulenken. Die Pfeile deuten die Ablenkrichtung des Partikelstrahls 16 in x-Richtung (horizontal) und in y-Richtung (vertikal) an. Somit ist die Rasterscaneinrichtung 44 in der Lage, eine zweidimensionale Matrix aus Punkten mit den Positionen $(x_j, y_j)$ zu scannen oder abzufahren. Diese Punkte $(x_j, y_j)$ werden in Kombination mit der jeweils verwendeten Partikelenergie als Scan Spots, Rasterpunkte oder Abtastpunkte bezeichnet. D.h. ein Abtastpunkt wird bestimmt durch die Ausrichtung des Partikelstrahls 16 in x-Richtung und y-Richtung, sowie durch die Partikelenergie. Für eine Kombination aus x- und y-Werten existieren demnach mehrere Abtastpunkte, wenn Partikel verschiedener Energien ausgesendet werden.

Das zu bestrahlende Zielvolumen 56 in dem zu bestrahlenden Patienten oder Objekt kann man als aus isoenergetischen Scheiben oder Schichten 58a, 58b, 58c, ... 58i zusammengesetzt betrachten. Hierbei sind die Iso-Energieschichten 58a, 58b, 58c, ... 58i jeweils einer bestimmten Position auf der z-Achse zugeordnet. Die Schichten werden als isoenergetisch bezeichnet, da Partikel einer bestimmten Anfangsenergie hauptsächlich mit der Materie der jeweiligen Schicht wechselwirken, d.h. die Energiedosis der Partikel dieser bestimmten Anfangsenergie wirkt sich zum großen Teil nur auf die jeweilige Iso-Energieschicht aus.

In dem Beispiel der Figur 2 beginnt die Zählung der Schichten an der der Rasterscaneinrichtung 44 zugewandten Seite mit 58a, während die von der Rasterscaneinrichtung 44 am weitesten entfernte Schicht, die distale Schicht, die Bezeichnung 58i hat, wobei i die Anzahl der Schichten bezeichnet. Zur Einstellung des Partikelstrahls 16 auf eine jeweiligen Schicht 58a, 58b, 58c, ... 58i weist der Partikelstrahl 16 jeweils eine andere Anfangsenergie auf, wobei die Anfangsenergie diejenige der Partikel vor der Wechselwirkung mit dem Objekt 14 oder 18 ist. Hierbei wird der Partikelstrahl 16 mit der niedrigsten Energie in der Scheibe 58a und der Partikelstrahl 16 mit der höchsten Energie in der Scheibe 58i deponiert.

Die Bestrahlung mit einem Scan-Verfahren bedient sich folglich eines Partikelstrahls 16, welcher so dimensioniert ist, dass in dem Zielvolumen 56 lediglich an einem kleinen, umschriebenen Bezirk eine Einzeldosis deponiert werden kann. Ein solcher kleiner Bezirk entspricht einem Zielpunkt, wobei die Koordinaten der Zielpunkte für die Bestrahlungsplanung bekannt sind. Dementsprechend kann durch Ansteuern eines bestimmten Abtastpunktes ein bestimmter Zielpunkt bestrahlt werden.

Um das gesamte Zielvolumen 56 zu bestrahlen, werden sukzessive die unterschiedlichen Abtastpunkte angesteuert. Der Partikelstrahl 16 wird dabei mithilfe der Scan-Magneten 46 und 48 abgelenkt und so über das Zielvolumen gelenkt. Zur Bestrahlung unterschiedlicher Iso-Energieschichten wird die Energie des Partikelstrahls 16 passend eingestellt. Gezeigt ist in Figur 2 ein Zielvolumen 56, bei dem drei distale Iso-Energieschichten $58_i$, $58_{i-1}$, $58_{i-2}$ bereits bestrahlt worden sind, und bei denen aktuell der Partikelstrahl 16 über die nachfolgenden Iso-Energieschicht $58_{i-3}$ scannt.

Vor der Bestrahlung eines Zielvolumens 56 wird eine Bestrahlungsplanung durchgeführt, um dann die Bestrahlung, d.h. das Scannen bzw. Abtasten des Zielvolumens 56 mit dem Partikelstrahl 16, entsprechend der erstellten Bestrahlungsplanung zu steuern. Die Bestrahlungsplanung stellt also die Bestimmung von Steuerparametern zur Steuerung der Bestrahlungsanlage 10 dar. Die Bestrahlungsplanung wird mittels einer Bestrahlungsplanungsvorrichtung 68 durchgeführt. Die Bestrahlungsplanungsvorrichtung 68 ist z.B. ein Arbeitsplatz-Computer, eine Workstation oder ein anderer Rechner. Die Bestrahlungsplanungsvorrichtung 68 bestimmt die Steuerparameter, die zur Steuerung der späteren Bestrahlung eingesetzt werden.

Die Steuerparameter werden zur Durchführung der Bestrahlung an die Bestrahlungsvorrichtung 10 weitergeleitet. Selbstverständlich muss die Bestrahlungsplanungsvorrichtung 68 nicht physikalisch an die Bestrahlungsvorrichtung 10 angebunden sein, wie in Figur 2 dargestellt. Vielmehr ist es beispielsweise auch möglich, dass die Berechnungsergebnisse der Bestrahlungsplanungsvorrichtung 68 über einen Datenträger zur Bestrahlungsvorrichtung 10 transferiert werden. Auch kann zwischen Planung und Bestrahlung ein gewisser Zeitraum, z.B. von mehreren Tagen, liegen.

Die Bestrahlungsvorrichtung 10 wird von einem Kontrollsystem gesteuert, das einzelne untergeordnete Steuerungseinrichtungen für verschiedene Teilsysteme umfasst. Hierzu gehören z.B. die Steuereinrichtung 66 für die Rasterscaneinrichtung 44, und eventuell weitere zusätzliche Steuereinrichtungen für andere Teile der Bestrahlungsvorrichtung 10, welche der Übersichtlichkeit halber nicht dargestellt sind. Das Kontrollsystem steuert dabei den Bestrahlungsverlauf entsprechend der von der Bestrahlungsplanungsvorrichtung 68 ermittelten Steuerparameter.

Zunächst wird mittels eines Computer-Tomographen oder Kernspin-Tomographen oder anderer diagnostischer Einrichtungen die Lage und Ausdehnung eines zu bestrahlenden Tumors oder eines anderen Zielvolumens 56 ermittelt. Die Bestrahlungsplanungsvorrichtung 68 erhält Daten dieser Bildgebungsvorrichtungen über einen Eingang 69.

Die Bestrahlungsplanungsvorrichtung 68 ist ferner durch ihre Benutzerschnittstelle 70 dazu ausgebildet, dass ein Anwender ihr das oder die Zielvolumen 56 und die zu applizierende Dosisverteilung vorgibt. Der Anwender kann also für die Zielpunkte des Zielvolumens 56 eine Energiedosis angeben, im folgenden als Zieldosisverteilung bezeichnet.

Die Bestrahlungsplanungsvorrichtung 68 ermittelt anhand der vorgegebenen Zieldosisverteilung die Steuerparameter für die Bestrahlung. Insbesondere ist zu ermitteln, wie viele Partikel einer bestimmten Energie für jeden Abtastpunkt auszustrahlen sind. Hierbei ist zu berücksichtigen, dass die Intensität des Partikelstrahls, d.h. die Anzahl der Teilchen pro Sekunde, innerhalb jeder Iso-Energieschicht konstant ist. Die Intensität muss daher an den Abtastpunkt mit der kleinsten Teilchenanzahl innerhalb der Iso-Energieschicht angepasst werden. Existieren innerhalb dieser Iso-Energieschicht Abtastpunkte mit einer deutlich höheren Teilchenzahl, so wird aufgrund der konstanten Intensität eine große Zeitdauer für die Bestrahlung dieser Abtastpunkte benötigt.

Für einen Patienten ist eine große Zeitdauer der Bestrahlung i.d.R. unangenehm. Dies ist insbesondere dadurch bedingt, dass der Patient während der Bestrahlung fixiert werden muss, so dass Bewegungen des Zielvolumens weitgehend ausgeschlossen sind. Um lange Bestrahlungsdauern zu vermeiden, sollte daher darauf geachtet werden, dass die Dynamik der Teilchenzahlen innerhalb jeder Iso-Energieschicht klein ist. Diese Dynamik kann auf verschiedene Weisen ausgedrückt werden, z.B. als das Verhältnis von größter zu kleinster Partikelanzahl der Abtastpunkte innerhalb der Iso-Energieschicht oder auch als der Abstand zwischen der größten und der kleinsten Partikelanzahl der Abtastpunkte innerhalb der Iso-Energieschicht, oder auch als Varianz der Partikelanzahlen innerhalb einer Iso-Energieschicht.

Bei der Bestrahlungsplanung sind daher bei der Ermittlung der Teilchenzahlen zwei gegenläufige Interessen vorhanden: zum einen eine möglichst gute Dosisverteilung, worunter eine Dosisverteilung möglichst nahe der vorgegebenen Zieldosisverteilung zu verstehen ist, und zum anderen eine geringe Teilchenzahldynamik innerhalb jeder Iso-Energieschicht, entsprechend einer geringen Bestrahlungsdauer für diese Iso-Energieschicht.

Es wird von der Bestrahlungsplanungsvorrichtung 68 ein Optimierungsverfahren zur Ermittlung der bei der Bestrahlung anzuwendenden Partikelanzahlen eingesetzt. Um die beiden genannten Größen zu berücksichtigen, wird hierzu die folgende Kostenfunktion F(I) eingesetzt:

$$F(I) = \sum_i \left[ s_i \left( \Delta d_i(I) \right)^2 \right] + \alpha \cdot R(I)$$

Hierbei ist $I=[I_1, I_2, ..., I_N]$ das N-Tupel der ermittelten Teilchenanzahlen, also die Partikelanzahlverteilung. Dies ist die Größe, welche es zu ermitteln gilt und welche als Ergebnis ausgegeben wird. $I_1$ ist hierbei die Anzahl der Partikel, die für den ersten Abtastpunkt anzuwenden sind, $I_2$ die Anzahl der Partikel, die für den zweiten Abtastpunkt anzuwenden sind, usw. Insgesamt sind für alle Iso-Energieschichten zusammen N Abtastpunkte vorhanden.

Die Kostenfunktion F ist eine Funktion der freien Optimierungsparameter, d.h. der Teilchenzahlen $I_i$ an allen Abtastpunkten i. Sie ist ein Maß für die negative Güte, also die "Schlechtigkeit" von I. Zur Ermittlung einer geeigneten Teilchenanzahlverteilung I wird die Kostenfunktion F(I) minimiert. Hierzu existieren bekannte Verfahren, z.B. beschrieben in Krämer et al.: Treatment Planning for heavy-ion Radiotherapy: physical beam model and dose optimization, Phys.Med.Biol. 45 (2000) 3299-3317.

Bezüglich des ersten Summanden der Kostenfunktion F(I) gilt folgendes:

Der Index i bezieht sich auf die Zielpunkte. Wurden beispielsweise für M Punkte Zieldosiswerte eingegeben, läuft i von 1 bis M.

$\Delta d_i(I)$ ist die Abweichung zwischen der Dosis des Zielpunktes i bei den ermittelten Partikelanzahlen $I=[I_1, I_2, ..., I_N]$ und der jeweiligen Zieldosis des Zielpunktes i. Es werden also die quadratischen Abweichungen der tatsächlichen zu den gewünschten Dosiswerten betrachtet.

Bei $s_i$ handelt es sich um Gewichtungsfaktoren. Hierdurch kann berücksichtigt werden, dass manche Zielpunkte hinsichtlich des Einhaltens der vorgegebenen Zieldosis wichtiger sind als andere. Befindet sich z.B. ein besonders empfindliches Organ, welches also einer möglichst geringen Dosis auszusetzen ist, innerhalb des Bestrahlungsvolumens, so kann dieses einen höheren Gewichtungsfaktor erhalten. Ein Beispiel hierfür wäre der Hirnstamm, für welchen von dem Nutzer z.B. eine Zieldosis von nahe Null vorgegeben sein könnte.

Der erläuterte erste Summand der Kostenfunktion berücksichtigt also, wie weit die ermittelte Dosisverteilung von der Zieldosisverteilung entfernt ist. Damit wird dem ersten der beiden oben genannten Ziele Rechnung, der möglichst guten Dosisverteilung, getragen.

Bezüglich des zweiten Summanden der Kostenfunktion F(I) gilt folgendes:

Bei R handelt es sich um einen Regularisierungsterm, welcher dem zweiten der oben genannten Ziele Rechnung trägt, nämlich der anzustrebenden geringen Teilchenzahldynamik. Für die Berechnung von R gibt es mehrere Möglichkeiten. Eine davon wird im Folgenden näher erläutert. Gemäß diesem Beispiel setzt sich R folgendermaßen zusammen:

$$R = \sum_E \beta(E) \sigma^2 \left( \frac{I_E}{\langle I_E \rangle} \right)$$

Der Index E steht hier für die Iso-Energieschicht. Zur Berechnung von R wird also über alle Iso-Energieschichten summiert.

$I_E$ ist dementsprechend ein Subtupel von I und enthält nur diejenigen Werte der Partikelanzahlverteilung I, welche zu den Abtastpunkten der jeweiligen Iso-Energieschicht E gehören.

Die Funktion $\sigma^2(x)$ berechnet die Varianz eines Tupels x, und $\langle x \rangle$ berechnet den Mittelwert der Elemente eines Tupels x der Größe M, d.h. $\langle x \rangle = \dfrac{1}{M} \sum_{i=1}^{M} x_i$ .

Um eine Unabhängigkeit von den absoluten Teilchenanzahlen zu erreichen, wird eine Normalisierung in Bezug auf den Mittelwert vorgenommen. Dies äußert sich darin, dass nicht die Varianz von $I_E$, sondern der normalisierten

Größe $\dfrac{I_E}{\langle I_E \rangle}$ berechnet wird.

Der Gewichtungsfaktor $\beta(E)$ wird bestimmt über

$$\beta(E) = \frac{Anzahl\ der\ Rasterpunkte\ in\ Iso-Energieschicht\ E}{gesamte\ Anzahl\ der\ Rasterpunkte\ N} .$$

Hierdurch erlangen Iso-Energieschichten mit wenigen Abtastpunkten eine niedrigere Bedeutung als Iso-Energieschichten mit vielen Abtastpunkten. Dies ist sinnvoll, da eine ungünstige, also hohe, Dynamik sich innerhalb einer großen Iso-Energieschicht viel stärker auf die zeitliche Länge der Bestrahlung auswirken kann als dies bei einer kleinen Iso-Energieschicht, also einer Iso-Energieschichten mit wenigen Abtastpunkten, möglich ist.

Der Regularisierungsterm besteht also aus einer gewichteten Summe über alle Iso-Energieschichten der normierten Varianzen der ermittelten Teilchenanzahlen. Die Varianz ist eine sinnvolle Größe zur Abschätzung der Dynamik, da sie ein Maß für die Streuung der Werte innerhalb einer Iso-Energieschicht ist. Der zweite Summand ergänzt die Kostenfunktion F(I) somit um einen Term, welcher eine hohe Teilchenzahldynamik bestraft. Hierdurch werden bei der Minimierung der Kostenfunktion Lösungen in Form von Teilchenanzahlverteilungen I begünstigt, welche sich durch geringe Dynamiken auszeichnen.

Bei $\alpha$ handelt es sich um einen Parameter größer oder gleich Null, welcher die beiden Summanden der Kostenfunktion F(I) relativ zueinander gewichtet. Dieser Parameter kann von dem Nutzer vorgegeben werden. So ist es beispielsweise möglich, dass der Nutzer eine bestimmte Zieldosisverteilung und einen bestimmten Wert für $\alpha$ vorgibt, woraufhin durch Minimierung der Kostenfunktion F(I) eine Partikelanzahlverteilung I ermittelt wird. Für dieses ermittelte I kann dem Nutzer im Anschluss angezeigt werden, wie nahe man bei Einsatz von I der Zieldosisverteilung kommt und wie lange für die Bestrahlung voraussichtlich benötigt wird. Stimmt der Nutzer diesem Ergebnis zu, kann die Bestrahlung entsprechend der ermittelten Teilchenanzahlverteilung I erfolgen. Um hingegen eine bessere Annäherung an die Zieldosisverteilung zu erreichen, kann der Nutzer den Wert für $\alpha$ verringern; um andererseits die Zeitdauer, welche für die Bestrahlung benötigt wird, zu reduzieren, kann der Nutzer den Wert für $\alpha$ erhöhen. Mit diesem neuen Wert für den Parameter $\alpha$ wird dann erneut durch Minimierung der neuen Kostenfunktion F(I) die Teilchenanzahlverteilung I ermittelt. Es ist also eine stufenlose Abwägung der beiden Optimierungsziele, d.h. der optimalen Dosisverteilung und der niedrigen Teilchenzahldynamik, gegeneinander möglich. Im folgenden werden mathematische Aspekte der Optimierung betrachtet. Für viele effizient Optimierungsmethoden, die so genannten gradientenbasierten Methoden, ist es nötig, die Ableitung von K nach I zu berechnen.

Es wird zunächst die Berechnung der Ableitung von $\sigma^2(I_E/\langle I_E \rangle)$ nach I erläutert. Hierzu wird zunächst nur eine einzelne Iso-Energieschicht betrachtet, so dass der Index E weggelassen werden kann.

Unter Verwendung von $\sigma^2(x)=\langle x^2 \rangle - \langle x \rangle^2$ bestimmt sich die Varianz der normalisierten Teilchenanzahl $\dfrac{I}{\langle I \rangle}$ als:

$$\sigma^2\left(\frac{I}{\langle I \rangle}\right) = \left\langle \left(\frac{I}{\langle I \rangle}\right)^2 \right\rangle - \left\langle \frac{I}{\langle I \rangle} \right\rangle^2 = \frac{\langle I^2 \rangle}{\langle I \rangle^2} - 1$$

Die Quadrierung eines Tupels wird hierbei definiert als dasjenige Tupel, welches die Quadrate der Elemente enthält: $I^2 \equiv [I_1^2, \ldots, I_N^2]$ . .

Leitet man die Varianz hinsichtlich $I_k$ ab, betrachtet man also die Veränderung der Varianz bei Änderung der Teilchenanzahl des Abtastpunktes k, erhält man:

$$\frac{\partial \sigma^2}{\partial I_k} = \frac{1}{\langle I \rangle^2} \cdot \frac{\partial \langle I^2 \rangle}{\partial I_k} + \langle I^2 \rangle \cdot \frac{\partial}{\partial I_k}\left(\frac{1}{\langle I \rangle^2}\right) = \frac{1}{\langle I \rangle^2} \cdot \frac{2I_k}{N} + \langle I^2 \rangle \cdot \frac{-2}{\langle I \rangle^3 N} = \frac{2}{N\langle I \rangle^2}\left(I_k - \frac{\langle I^2 \rangle}{\langle I \rangle}\right).$$

Es ist günstig, als Optimierungsparmter nicht I, sondern w zu verwenden, wobei $w^2 = I$. Hierdurch wird ausgeschlossen, dass das ermittelte Ergebnis für I negative Teilchenanzahlen enthält. Die Ableitung wird somit zu:

$$\frac{\partial \sigma^2}{\partial w_k} = 2w_k \cdot \frac{\partial \sigma^2}{\partial I_k} = \frac{4w_k}{N\langle I \rangle^2}\left(I_k - \frac{\langle I^2 \rangle}{\langle I \rangle}\right).$$

Somit ergibt sich die Ableitung des gesamten Regularisierungsterms R zu:

$$\frac{\partial R}{\partial w_k} = \frac{\partial}{\partial w_k}\sum_E \beta(E)\sigma^2\left(\frac{I_E}{\langle I_E \rangle}\right) = \frac{\partial}{\partial w_k}\beta(E')\sigma^2\left(\frac{I_{E'}}{\langle I_{E'} \rangle}\right) = \beta(E')\frac{4w_k}{N_{E'}\langle I_{E'} \rangle^2}\left(I_k - \frac{\langle I_{E'}^2 \rangle}{\langle I_{E'} \rangle}\right)$$

Hierbei wurde berücksichtigt, dass sich der Abtastpunkt k innerhalb der Iso-Energieschicht E' befindet. Man kann dementsprechend ausnutzen, dass die Ableitung der Summe nur für den Summanden der Iso-Energieschicht E' ungleich Null ist. $N_{E'}$ ist die Anzahl der Abtastpunkte innerhalb der Iso-Energieschicht E' .

Die Ableitung der gesamten Kostenfunktion F(I) ist dann:

$$\frac{\partial F(I)}{\partial w_k} = \frac{\partial}{\partial w^k}\left[\sum_i s_i\left(\Delta d_i(I)\right)^2\right] + \alpha\beta(E')\frac{4w_k}{N_{E'}\langle I_{E'} \rangle^2}\left(I_k - \frac{\langle I_{E'}^2 \rangle}{\langle I_{E'} \rangle}\right),$$

wobei der Abtastpunkt k sich in der Iso-Energieschicht E' befindet.

Unter Verwendung dieser Ableitung wird die Kostenfunktion F(I) nun minimiert. Bei einer iterativen Vorgehensweise wird für ein aktuelles Tupel I(t) mithilfe des Funktionswertes und den Ableitungen von K der nächst bessere Lösungskandidat I(t+1) ausgerechnet.

Die Randbedingung einer geringen Teilchenanzahldynamik fließt in die Optimierung der Teilchenanzahl I ein, so dass diese Randbedingung nicht nach einer erfolgten Ermittlung von I im Nachhinein zusätzlich berücksichtigt werden muss. Ein solches Vorgehen, bei welchem die Teilchenanzahlverteilung I ohne Berücksichtigung der Dynamik optimiert wird, wonach im Anschluss eine Anpassung von I an die gewünschte geringe Dynamik erfolgt, führt nämlich zu deutlichen Verschlechterungen der Dosisverteilung.

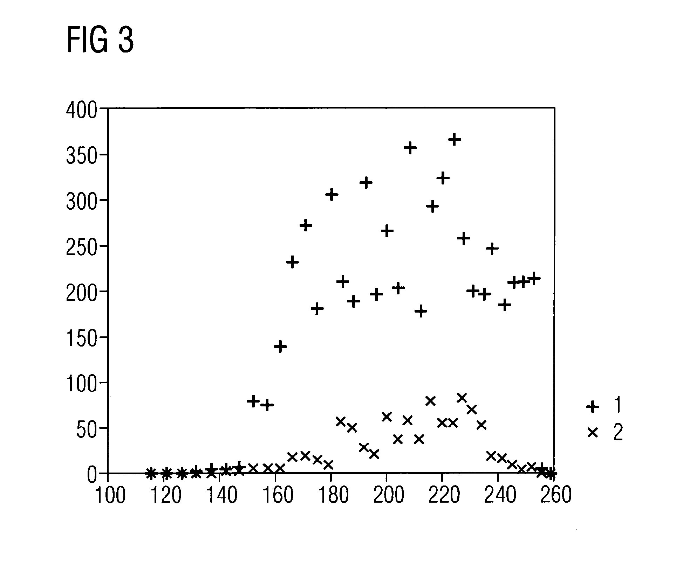

Figur 3 zeigt beispielhaft den vorteilhaften Effekt des beschriebenen Vorgehens für eine bestimmte vorgegebene Zieldosisverteilung. Auf der y-Achse ist hierbei die Teilchenanzahldynamik aufgetragen, wobei diese als Teilchenzahlhub, d.h. als Verhältnis der höchsten Teilchenanzahl zur niedrigsten Teilchenanzahl innerhalb einer Iso-Energieschicht, berechnet wurde. Auf der x-Achse sind die verschiedenen Iso-Energieschichten bzw. die hierzu gehörigen Anfangsenergien der Partikel in MeV aufgetragen. Die Kurve 1, gehörig zu den Symbolen +, entspricht einer ermittelten Teilchenanzahlverteilung I durch Optimierung ohne Berücksichtigung der Dynamik. Die Kurve 2, gehörig zu den Symbolen x, entspricht einer ermittelten Teilchenanzahlverteilung I durch Optimierung auf die oben beschriebene Weise mit Berücksichtigung der Dynamik. Es ist deutlich zu erkennen, dass die Kurve 2 wesentlich bessere Dynamik-Werte aufweist und somit zu einer reduzierten Bestrahlungsdauer führt.

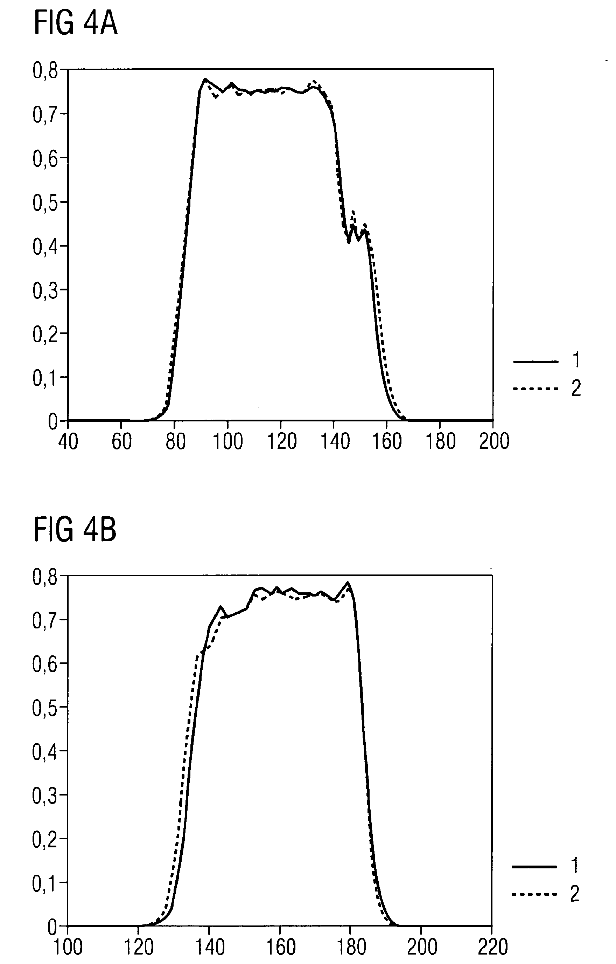

Anhand der Figuren 4A und 4B ist zu erkennen, dass die Dosisverteilung resultierend aus der Teilchenzahlverteilung der Kurve 2 aus Figur 3 nicht schlechter ist als diejenige der Kurve 1 aus Figur 3. Auf den y-Achsen ist hierbei jeweils die Dosis in Gray aufgetragen, auf der x-Achse eine Schnittlinie entlang des bestrahlten Objektes, d.h. eine Distanz in mm. Figur 4A und 4B entsprechen zwei zueinander senkrechten Schnitten durch eine auf ein Schädel-Chordom (ein bestimmter Tumortypus) optimierte Dosisverteilung. Die durchgezogene Kurve 1 entspricht wie auch die Kurve 1 der Figur 3 einer ermittelten Teilchenanzahlverteilung I durch Optimierung ohne Berücksichtigung der Dynamik. D.h. die Kurve 1 entspricht recht genau der vorgegebenen Zieldosisverteilung. Die gestrichelte Kurve 2 entspricht wie auch die Kurve 2 der Figur 3 einer ermittelten Teilchenanzahlverteilung I durch Optimierung auf die oben beschriebene Weise mit Berücksichtigung der Dynamik. Es ist deutlich zu erkennen, dass die beiden Kurven nicht wesentlich voneinander abweichen, dass also die Kurve 2 eine ähnlich gute Dosisverteilung liefert wie die Kurve 1. Durch Berücksichtigung der Teilchenzahldynamik und somit der Bestrahlungsdauer wird also keine Verschlechterung hinsichtlich der Dosisverteilung bewirkt.

Die Erfindung wurde voranstehend an einem Ausführungsbeispiel beschrieben. Es versteht sich, dass zahlreiche Änderungen und Modifikationen möglich sind, ohne dass der Rahmen der Erfindung verlassen wird.

**Patentansprüche**

1. Vorrichtung (68) zur Bestimmung von Steuerparametern für eine Partikelbestrahlungsanlage,
   wobei mit der Partikelbestrahlungsanlage verschiedene Dosiswerte an unterschiedlichen Zielpunkten in einem Zielvolumen (56) deponierbar sind, indem von einem Partikelstrahl Abtastpunkte angesteuert werden, wobei das Zielvolumen aus Iso-Energieschichten zusammengesetzt ist, und wobei ein Partikelstrahl (16) zur Einstellung auf eine jeweilige Iso-Energieschicht jeweils eine andere Energie aufweist,
   umfassend:

   einen Eingang (70) zum Empfangen von Informationen betreffend eine vorgegebene Dosis-Verteilung über Zielpunkte,
   einen Ermittlungsbestandteil zum Ermitteln einer bei der Bestrahlung zu deponierenden Partikelanzahlverteilung über Abtastpunkte,
   wobei die Ermittlung erfolgt unter Verwendung der vorgegebenen Dosis-Verteilung und einer Größe, welche Unterschiede der Partikelanzahlverteilung zwischen Partikelanzahlen verschiedener Abtastpunkte, die gemäß der Partikelanzahlverteilung mit Partikeln der gleichen Energie zu bestrahlen sind, berücksichtigt.

2. Vorrichtung (68) nach Anspruch 1, wobei
   die Größe Unterschiede zwischen Partikelanzahlen aller Abtastpunkte, die gemäß der Partikelanzahlverteilung mit Partikeln der gleichen Energie zu bestrahlen sind, berücksichtigt.

3. Vorrichtung (68) nach einem der Ansprüche 1 bis 2, wobei die Größe eine Streuung von Partikelanzahlen verschiedener Abtastpunkte, die gemäß der Partikelanzahlverteilung mit Partikeln der gleichen Energie zu bestrahlen sind, enthält.

4. Vorrichtung (68) nach einem der Ansprüche 1 bis 3, wobei die Größe eine Varianz von Partikelanzahlen verschiedener Abtastpunkte, die gemäß der Partikelanzahlverteilung mit Partikeln der gleichen Energie zu bestrahlen sind, enthält.

5. Vorrichtung (68) nach einem der Ansprüche 1 bis 4, wobei die Größe einen Wertebereich von Partikelanzahlen verschiedener Abtastpunkte, die gemäß der Partikelanzahlverteilung mit Partikeln der gleichen Energie zu bestrahlen sind, enthält.

6. Vorrichtung (68) nach einem der Ansprüche 1 bis 5, wobei die Größe die maximale Differenz von Partikelanzahlen aller Abtastpunkte, die gemäß der Partikelanzahlverteilung mit Partikeln der gleichen Energie zu bestrahlen sind, enthält.

7. Vorrichtung (68) nach einem der Ansprüche 1 bis 6, wobei die Größe das Verhältnis zwischen der höchsten und der niedrigsten Partikelanzahl aller Abtastpunkte, die gemäß der Partikelanzahlverteilung mit Partikeln der gleichen Energie zu bestrahlen sind, enthält.

8. Vorrichtung (68) nach einem der Ansprüche 1 bis 7, wobei die Ermittlung durch Optimierung einer Kostenfunktion erfolgt, und

   die Kostenfunktion einen ersten Term betreffend die vorgegebene Dosis-Verteilung und einen zweiten Term betreffend die Größe enthält.

9. Vorrichtung (68) nach Anspruch 8, wobei
   die Kostenfunktion einen vorgebbaren Parameter enthält zur relativen Wichtung des ersten gegenüber dem zweiten Term.

10. Vorrichtung (68) nach Anspruch 8 oder 9, wobei
    durch die Optimierung der Kostenfunktion die Partikelanzahlverteilung einerseits hinsichtlich der vorgegebenen Dosis-Verteilung und andererseits hinsichtlich einer Bestrahlungs-Zeitdauer optimiert wird.

11. Partikelbestrahlungsanlage mit einer Vorrichtung (68) zur
    Bestimmung von Steuerparametern nach einem der Ansprüche 1 bis 10.

12. Verfahren zum Bestimmen von Steuerparametern für eine
    Partikelbestrahlungsanlage, wobei mit der Partikelbestrahlungsanlage verschiedene Dosiswerte an unterschiedlichen Zielpunkten in einem Zielvolumen (56) deponierbar sind, indem von einem Partikelstrahl Abtastpunkte angesteuert werden, wobei das Zielvolumen aus Iso-Energieschichten zusammengesetzt ist, und wobei ein Partikelstrahl (16) zur Einstellung auf eine jeweilige Iso-Energieschicht jeweils eine andere Energie aufweist,
    mit folgenden Schritten:

    Empfangen von Informationen betreffend eine vorgegebene Dosis-Verteilung über Zielpunkte,
    Ermitteln einer bei der Bestrahlung zu deponierenden Partikelanzahlverteilung über Abtastpunkte,
    wobei die Ermittlung erfolgt unter Verwendung der vorgegebenen Dosis-Verteilung und einer Größe, welche Unterschiede der Partikelanzahlverteilung zwischen Partikelanzahlen verschiedener Abtastpunkte, die gemäß der Partikelanzahlverteilung mit Partikeln der gleichen Energie zu bestrahlen sind, berücksichtigt.

13. Verfahren zur Bestrahlung eines Zielvolumens (56) mit
    Partikeln unter Verwendung von Steuerparametern zur Steuerung einer Partikelbestrahlungsanlage, wobei die Steuerparameter mit einem Verfahren nach Anspruch 12 ermittelt sind, wobei das Zielvolumen (56) zumindest einen Teilbereich eines nicht-lebenden Körpers, insbesondere eines Phantoms, zur Überprüfung einer Bestrahlungsplanung umfasst.

14. Computerprogramm mit Programmcode-Mitteln, um das Verfahren nach einem der Ansprüche 12 bis 13 durchzuführen, wenn das Computerprogramm auf einem Computer ausgeführt wird.

15. Computerprogrammprodukt, umfassend auf einem computerlesbaren Datenträger gespeicherte Programmcode-Mittel eines Computerprogramms,
    um das Verfahren nach einem der Ansprüche 12 bis 13 durchzuführen, wenn das Computerprogramm auf einem Computer ausgeführt wird.

**Claims**

1. Device (68) for determining control parameters for a particle irradiation system,
   wherein different dose values can be deposited at different target points in a target volume (56) by means of the particle irradiation system through selection of sampling points by a particle beam, wherein the target volume is composed of iso-energy layers, and wherein a particle beam (16) has a different energy in each case for setting to a respective iso-energy layer,
   the device comprising:

   an input (70) for receiving information relating to a predefined dose distribution over target points,
   a determination component for determining a particle number distribution to be deposited during the irradiation over sampling points,

wherein the determining takes place using the predefined dose distribution and a variable which takes into account differences in the particle number distribution between particle numbers of different sampling points which, according to the particle number distribution, are to be irradiated with particles of the same energy.

2. Device (68) according to claim 1, wherein
   the variable takes into account differences between particle numbers of all sampling points which, according to the particle number distribution, are to be irradiated with particles of the same energy.

3. Device (68) according to one of claims 1 to 2, wherein
   the variable includes a scatter of particle numbers of different sampling points which, according to the particle number distribution, are to be irradiated with particles of the same energy.

4. Device (68) according to one of claims 1 to 3, wherein the variable contains a variance of particle numbers of different sampling points which, according to the particle number distribution, are to be irradiated with particles of the same energy.

5. Device (68) according to one of claims 1 to 4, wherein the variable contains a value range of particle numbers of different sampling points which, according to the particle number distribution, are to be irradiated with particles of the same energy.

6. Device (68) according to one of claims 1 to 5, wherein the variable contains the maximum difference of particle numbers of all sampling points which, according to the particle number distribution, are to be irradiated with particles of the same energy.

7. Device (68) according to one of claims 1 to 6, wherein the variable contains the ratio between the highest and the lowest particle number of all sampling points which, according to the particle number distribution, are to be irradiated with particles of the same energy.

8. Device (68) according to one of claims 1 to 7, wherein the determining takes place through optimisation of a cost function, and
   the cost function contains a first term relating to the predefined dose distribution and a second term relating to the variable.

9. Device (68) according to claim 8, wherein
   the cost function contains a predefinable parameter for relative weighting of the first term with respect to the second term.

10. Device (68) according to claim 8 or 9, wherein
    by means of the optimisation of the cost function the particle number distribution is optimised on the one hand in respect of the predefined dose distribution and on the other hand in respect of an irradiation duration.

11. Particle irradiation system having a device (68) for
    determining control parameters according to one of claims 1 to 10.

12. Method for determining control parameters for a particle
    irradiation system, wherein different dose values can be deposited at different target points in a target volume (56) by means of the particle irradiation system through selection of sampling points by a particle beam, wherein the target volume is composed of iso-energy layers, and wherein a particle beam (16) has a different energy in each case for setting to a respective iso-energy layer, the method comprising the following steps of:

    receiving information relating to a predefined dose distribution via target points,
    determining a particle number distribution to be deposited during the irradiation via sampling points,
    wherein the determining takes place using the predefined dose distribution and a variable which takes into account differences in the particle number distribution between particle numbers of different sampling points which,
    according to the particle number distribution, are to be irradiated with particles of the same energy.

13. Method for irradiating a target volume (56) with particles using control parameters for controlling a particle irradiation system, wherein the control parameters are determined by means of a method according to claim 12, and wherein the target volume (56) comprises at least one subregion of a non-living body, in particular a phantom, for the purpose of verifying an irradiation plan.

14. Computer program having program code means for the purpose of performing the method according to one of claims 12 to 13 when the computer program is executed on a computer.

15. Computer program product comprising program code means of a computer program which are stored on a computer-readable data medium for the purpose of performing the method according to one of claims 12 to 13 when the computer program is executed on a computer.

**Revendications**

1. Dispositif (68) pour déterminer des paramètres de commande pour une installation d'irradiation particulaire, l'installation d'irradiation particulaire permettant de déposer différentes valeurs de dose à différents points cibles dans un volume cible (56) par le fait qu'un rayon particulaire se dirige sur des points de balayage, le volume cible étant composé de couches isoénergétiques et un rayon particulaire (16) présentant respectivement une autre énergie pour réglage sur une couche isoénergétique respective, comprenant :

   - une entrée (70) pour recevoir des informations concernant une distribution prédéterminée de la dose sur des points cibles ; - un composant de détermination pour déterminer une distribution, sur des points de balayage, du nombre de particules à déposer lors de l'irradiation ;
   la détermination s'effectuant avec utilisation de la distribution prédéterminée de la dose et d'une grandeur qui tient compte de différences de la distribution du nombre de particules entre des nombres de particules de différents points de balayage à irradier avec des particules de même énergie conformément à la distribution du nombre de particules.

2. Dispositif (68) selon la revendication 1, la grandeur tenant compte de différences entre des nombres de particules de tous les points de balayage à irradier avec des particules de même énergie conformément à la distribution du nombre de particules.

3. Dispositif (68) selon l'une des revendications 1 à 2, la grandeur contenant une dispersion de nombres de particules de différents points de balayage à irradier avec des particules de même énergie conformément à la distribution du nombre de particules.

4. Dispositif (68) selon l'une des revendications 1 à 3, la grandeur contenant une variance de nombres de particules de différents points de balayage à irradier avec des particules de même énergie conformément à la distribution du nombre de particules.

5. Dispositif (68) selon l'une des revendications 1 à 4, la grandeur contenant une plage de valeurs de nombres de particules de différents points de balayage à irradier avec des particules de même énergie conformément à la distribution du nombre de particules.

6. Dispositif (68) selon l'une des revendications 1 à 5, la grandeur contenant la différence maximale de nombres de particules de tous les points de balayage à irradier avec des particules de même énergie conformément à la distribution du nombre de particules.

7. Dispositif (68) selon l'une des revendications 1 à 6, la grandeur contenant le rapport entre le nombre de particules le plus élevé et le nombre de particules le plus bas de tous les points de balayage à irradier avec des particules de même énergie conformément à la distribution du nombre de particules.

8. Dispositif (68) selon l'une des revendications 1 à 7, la détermination s'effectuant par optimisation d'une fonction de coût et la fonction de coût contenant un premier terme relatif à la distribution prédéterminée de la dose et un deuxième terme relatif à la grandeur.

9. Dispositif (68) selon la revendication 8, la fonction de coût contenant un paramètre prédéterminable pour la pondération relative du premier terme par rapport au second.

10. Dispositif (68) selon la revendication 8 ou 9, l'optimisation de la fonction de coût permettant l'optimisation de la distribution du nombre de particules, d'une part en termes de distribution prédéterminée de la dose et, d'autre part, en termes de durée d'irradiation.

11. Installation d'irradiation particulaire avec un dispositif (68) pour déterminer des paramètres de commande selon l'une des revendications 1 à 10.

12. Procédé pour déterminer des paramètres de commande pour une installation d'irradiation particulaire, l'installation d'irradiation particulaire permettant de déposer différentes valeurs de dose à différents points cibles dans un volume cible (56) par le fait qu'un rayon particulaire se dirige sur des points de balayage, le volume cible étant composé de couches isoénergétiques et un rayon particulaire (16) présentant respectivement une autre énergie pour réglage sur une couche isoénergétique respective, comprenant les étapes suivantes :

   - réception d'informations concernant une distribution prédéterminée de la dose sur des points cibles ;
   - détermination d'une distribution, sur des points de balayage, du nombre de particules à déposer lors de l'irradiation ;
   la détermination s'effectuant avec utilisation de la distribution prédéterminée de la dose et d'une grandeur qui tient compte de différences de la distribution du nombre de particules entre des nombres de particules de différents points de balayage à irradier avec des particules de même énergie conformément à la distribution du nombre de particules.

13. Procédé d'irradiation d'un volume cible (56) avec des particules avec utilisation de paramètres de commande pour commander une installation d'irradiation particulaire, les paramètres de commande étant déterminés avec un procédé selon la revendication 12, le volume cible (56) comprenant au moins une zone partielle d'un corps non vivant, et plus particulièrement d'un fantôme, pour vérifier une planification d'irradiation.

14. Programme informatique avec des moyens de code de programme pour exécuter le procédé selon l'une des revendications 12 à 13 lorsque le programme informatique est exécuté sur un ordinateur.

15. Produit de programme informatique, comprenant des moyens de code de programme d'un programme informatique stockés sur un support de données lisible par un ordinateur pour exécuter le procédé selon l'une des revendications 12 à 13 lorsque le programme informatique est exécuté sur un ordinateur.

FIG 1

FIG 2

# FIG 3

FIG 4A

FIG 4B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1818078 A

- US 20070201614 A

**In der Beschreibung aufgeführte Nicht-Patentliteratur**

- *Phys. Med. Biol.,* vol. 52, 5973-5984

- **Krämer et al.** Treatment Planning for heavy-ion Radiotherapy: physical beam model and dose optimization. *Phys.Med.Biol.,* 2000, vol. 45, 3299-3317